# EUROPEAN PATENT APPLICATION

(11) **EP 4 670 665 A2**
(43) Date of publication of application: **31.12.2025**
(21) Application number: 25201729.8
(22) Date of filing: 27.07.2022
(51) Int. Cl.: A61B 34/20

(54) **ELECTRICAL FIELD VISUALIZATION FOR ELECTROPORATION CATHETER WITH MULTIPLE STATES**

(30) Priority: 27.07.2021 US 202163226075 P
(62) Divisional of application: 22757744.2
(71) Applicant: Boston Scientific Scimed, Inc., Maple Grove, MN 55311 (US)
(72) Inventor: GUTBROD, Sarah, R., St. Paul, MN, 55101 (US); KOOP, Brendan, E., Ham Lake, MN, 55304 (US); OLYNYK, Dustin, J., Eagan, MN, 55123 (US); GUGGENBERGER, Kurt, Edmund, North Andover, MA, 01845 (US); KAUPHUSMAN, James, Champlin, MN, 55316 (US); MAIERHOFER, Edward, J., Brooklyn, MN, 55443 (US); DAABOUL, Lauren, Maple Grove, MN, 55311 (US); DEDKOV, Dmitriy, Maple Grove, MN, 55311 (US); DIMITROV, Petar, Maple Grove, MN, 55311 (US)
(74) Representative: Pfenning, Meinig & Partner mbB

(57) **Abstract**

A system for electroporation ablation including a catheter having an electrode assembly and one or more states. The electrode assembly may be in different shapes when the catheter is at different states. The controller is configured to generate, based on one or more models of electric fields, graphical representations of electric fields generated by the electrode assembly when the catheter is at different states. In some embodiments, the controller is configured to overlay the graphical representations of the one or more electric fields on an anatomical map of a patient.

## Description

### CROSS-REFERENCE TO RELATED APPLICATION

This application claims priority to U.S. Provisional Application No. 63/226,075 filed July 27, 2021, the entire contents of which are incorporated by reference in its entirety.

### TECHNICAL FIELD

The present disclosure relates to medical systems and methods for ablating tissue in a patient. More specifically, the present disclosure relates to medical systems and methods for ablation of tissue by electroporation.

### BACKGROUND

Ablation procedures are used to treat many different conditions in patients. Ablation can be used to treat cardiac arrhythmias, benign tumors, cancerous tumors, and to control bleeding during surgery. Usually, ablation is accomplished through thermal ablation techniques including radio-frequency (RF) ablation and cryoablation. In RF ablation, a probe is inserted into the patient and radio frequency waves are transmitted through the probe to the surrounding tissue. The radio frequency waves generate heat, which destroys surrounding tissue and cauterizes blood vessels. In cryoablation, a hollow needle or cryoprobe is inserted into the patient and cold, thermally conductive fluid is circulated through the probe to freeze and kill the surrounding tissue. RF ablation and cryoablation techniques indiscriminately kill tissue through cell necrosis, which may damage or kill otherwise healthy tissue, such as tissue in the esophagus, phrenic nerve cells, and tissue in the coronary arteries.

Another ablation technique uses electroporation. In electroporation, or electro-permeabilization, an electrical field is applied to cells in order to increase the permeability of the cell membrane. The electroporation can be reversible or irreversible, depending on the strength of the electric field. If the electroporation is reversible, the increased permeability of the cell membrane can be used to introduce chemicals, drugs, and/or deoxyribonucleic acid (DNA) into the cell, prior to the cell healing and recovering. If the electroporation is irreversible, the affected cells are killed through apoptosis.

Irreversible electroporation can be used as a nonthermal ablation technique. In irreversible electroporation, trains of short, high voltage pulses are used to generate electric fields that are strong enough to kill cells through apoptosis. In ablation of cardiac tissue, irreversible electroporation can be a safe and effective alternative to the indiscriminate killing of thermal ablation techniques, such as RF ablation and cryoablation. Irreversible electroporation can be used to kill targeted tissue, such as myocardium tissue, by using an electric field strength and duration that kills the targeted tissue but does not permanently damage other cells or tissue, such as non-targeted myocardium tissue, red blood cells, vascular smooth muscle tissue, endothelium tissue, and nerve cells. Planning irreversible electroporation ablation procedures can be difficult due to the lack of acute visualization or data indicating which tissues have been irreversibly electroporated, as opposed to reversibly electroporated. Where tissue recovery can occur over minutes, hours, or days after the ablation is completed.

### SUMMARY

In Example 1, a system for electroporation ablation comprises a catheter including an electrode assembly and a plurality of states, the electrode assembly having a first shape when the catheter is at a first state of the plurality of states, the electrode assembly having a second shape when the catheter is at a second state of the plurality of states. The second shape may be different from the first shape, and the electrode assembly may include a plurality of electrodes. The system for electroporation ablation further comprises a controller configured to generate, based on a first model of electric fields, a first graphical representation of first electric fields generated by the plurality of the electrodes when the catheter is at the first state and deployed proximate to a target location. The controller may be further configured to generate, based on a second model of electric fields, a second graphical representation of second electric fields generated by the plurality of the electrodes when the catheter is at the second state and deployed proximate to the target location, and overlay, in a graphical user interface, the first graphical representations of the first electric fields and the second graphical representations of the second electric fields on an anatomical map of a patient proximate to the target location.

In Example 2, the system of Example 1, wherein the second shape is dissimilar to the first shape.

In Example 3, the system of Example 1, wherein the second shape is smaller than the first shape in volume.

In Example 4, the system of any of Examples 1-3, wherein the catheter includes a catheter shaft defining a longitudinal axis, wherein the electrode assembly includes a plurality of splines, a proximal end and a distal end, wherein at least a part of the plurality of electrodes are disposed on the plurality of splines, wherein the proximal end of the electrode assembly extends from the catheter shaft.

In Example 5, the system of Example 4, wherein each spline of the plurality of splines is arranged in a curve around the longitudinal axis and between the distal end and the proximal end of the electrode assembly when the catheter is at the first state.

In Example 6, the system of Example 4, wherein the plurality of splines are arranged in petal-like curves when the catheter is at the second state.

In Example 7, the system of any of Examples 1-6, wherein the controller is further configured to generate an indication of a difference between the first graphical representation of the first electric fields and the second graphical representation of the second electric fields.

In Example 8, the system of any of Examples 1-7, wherein the first graphical representation of the first electric fields includes one or more first areas, wherein field strength of the first electric fields within the one or more first areas is greater than a predefined threshold in magnitude.

In Example 9, the system of Example 8, wherein the second graphical representation of the second electric fields includes one or more second areas, wherein field strength of the second electric fields within the one or more second area is greater than the predefined threshold in magnitude.

In Example 10, the system of any of Examples 1-9, wherein the first graphical representation includes a first representation of the catheter.

In Example 11, the system of any of Examples 1-10, wherein the controller is further configured to generate, a software widget including a second representation of the catheter and an indication of one or more therapeutic sessions of electroporation ablation conducted by the catheter, and to present the software widget in the graphical user interface. In some instances, the software widget includes an indication identifying a therapeutic session of the one or more therapeutic sessions.

In Example 12, a method of planning ablation by electroporation comprises generating, by a controller and based on a first model of electric fields, a first graphical representation of first electric fields produced using electrodes on a catheter at a first state, the catheter including an electrode assembly having a first shape when the catheter is at the first state; presenting, on a display, the first graphical representation of the electric fields and an anatomical map of a patient proximate to a target location; generating, by a controller and based on a second model of electric fields, a second graphical representation of second electric fields produced using electrodes on a catheter at a second state, the catheter including an electrode assembly having a second shape when the catheter is at the second state, the second shape being different from the first shape; and presenting, on the display, the second graphical representation of the electric fields and the anatomical map proximate to the target location.

In Example 13, the method of Example 12, wherein the second shape is dissimilar to the first shape.

In Example 14, the method of either Example 12 or 13, further comprising generating an indication of a difference between the first graphical representation of the first electric fields and the second graphical representation of the second electric fields.

In Example 15, the method of any of Examples 12-14, further comprising generating a software widget including a second representation of the catheter and an indication of one or more therapeutic sessions of electroporation ablation conducted by the catheter, and presenting the software widget on the display.

In Example 16, a system for electroporation ablation comprises a catheter having a plurality of electrodes and a controller configured to generate a software widget including a representation of the catheter and an indication of one or more therapeutic sessions of electroporation ablation conducted by the catheter, and to present the software widget in a graphical user interface. In some instances, the software widget includes an indication identifying a therapeutic session of the one or more therapeutic sessions.

In Example 17, the system of Example 16, wherein the controller is further configured to generate, based on a model of electric fields, a graphical representation of the electric fields of the plurality of electrodes, and display the graphical representation of the electric fields of the plurality of electrodes.

In Example 18, the system of either Example 16 or 17, wherein the software widget includes a cross-sectional view of the catheter.

In Example 19, the system of any of Examples 16-18, wherein the software widget includes an alignment indicator representing axial relation between a catheter axis of the catheter and a target axis of a target ablation area of the electroporation ablation.

In Example 20, the system of any of Examples 16-19, wherein the representation the catheter includes a first representation of the catheter at a first time and a second representation of the catheter of the second time.

In Example 21, the system of Example 20, wherein the first representation and the second representation show a difference between the catheter at the first time and the catheter at the second time, wherein the difference includes at least one of a difference in shape, a difference in a rotating angle, a difference in electric field strength, and a difference in location.

In Example 22, the system of any of Examples 16-21, wherein the software widget further includes a field indication representing electric fields generated by the plurality of electrodes.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a diagram illustrating an exemplary clinical setting for treating a patient, and for treating a heart of the patient, using an electrophysiology system, in accordance with embodiments of the subject matter of the disclosure.
FIGS. 2A-2B are schematic views illustrating catheters that can be used for electroporation, including ablation by irreversible electroporation, in accordance with embodiments of the subject matter of the disclosure.
FIGS. 3A-3C are schematic views illustrating catheters that can be used for electroporation, including ablation by irreversible electroporation, in accordance with embodiments of the subject matter of the disclosure.
FIGS. 4A-4B are diagrams illustrating examples of graphical user interfaces, in accordance with embodiments of the subject matter of the disclosure.
FIGS. 5A-5C illustrate software widgets to facilitate planning and/or conducting ablation treatment, in accordance with embodiments of the subject matter of the disclosure.
FIGS. 6A-6B are diagrams illustrating a software widget and graphical representations of electric fields displayed side-by-side, in accordance with embodiments of the subject matter of the disclosure.
FIGS. 7A-7B illustrate examples of electric fields with various strength generated by a catheter including an electrode assembly.
FIG. 8 is flow chart diagram illustrating a method of planning ablation by irreversible electroporation, in accordance with embodiments of the subject matter of the disclosure.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

The following detailed description is exemplary in nature and is not intended to limit the scope, applicability, or configuration of the invention in any way. Rather, the following description provides some practical illustrations for implementing exemplary embodiments of the present invention. Examples of constructions, materials, and/or dimensions are provided for selected elements. Those skilled in the art will recognize that many of the noted examples have a variety of suitable alternatives.

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although illustrative methods may be represented by one or more drawings (e.g., flow diagrams, communication flows, etc.), the drawings should not be interpreted as implying any requirement of, or particular order among or between, various steps disclosed herein. However, certain some embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

Irreversible electroporation (IRE) uses high voltage, short (e.g., 100 microseconds or shorter) pulses to kill cells through apoptosis. IRE can be targeted to kill myocardium, sparing other adjacent tissues including the esophageal vascular smooth muscle and endothelium. IRE treatment can be delivered in multiple therapy sections. A therapy section (e.g., for a duration of 10 milliseconds) may include a plurality of electrical pulses (e.g., 20 pulses, 30 pulses, etc.) generated and delivered by an electroporation device, which is powered by an electroporation generator.

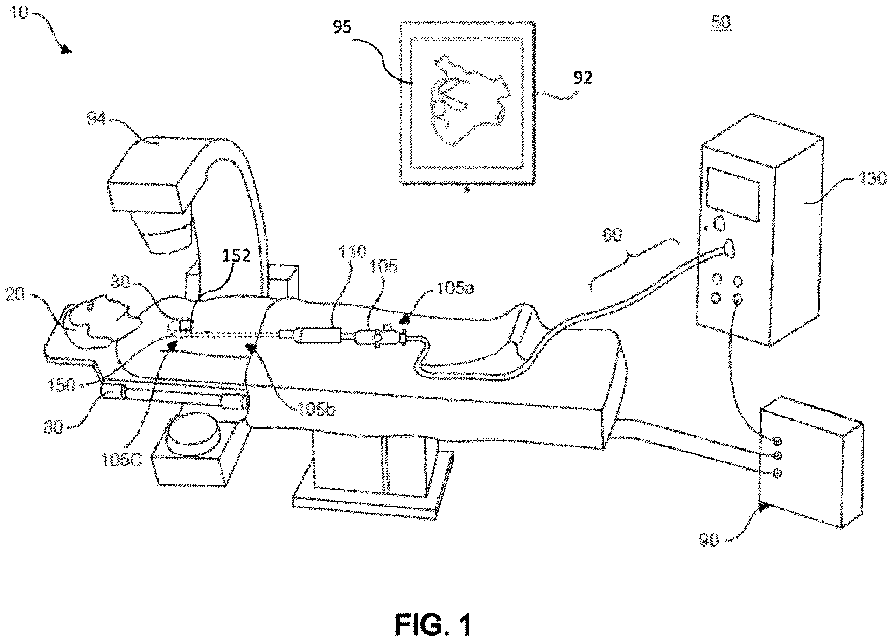

FIG. 1 is a diagram illustrating an exemplary clinical setting 10 for treating a patient 20, and for treating a heart 30 of the patient 20, using an electrophysiology system 50, in accordance with embodiments of the subject matter of the disclosure. The electrophysiology system 50 includes an electroporation device 60, a display 92, and an optional localization field generator 80. Also, the clinical setting 10 includes additional equipment such as imaging equipment 94 (represented by the C-arm) and various controller elements configured to allow an operator to control various aspects of the electrophysiology system 50. As will be appreciated by the skilled artisan, the clinical setting 10 may have other components and arrangements of components that are not shown in FIG. 1.

The electroporation device 60 includes an electroporation catheter 105, an introducer sheath 110, a controller 90, and an electroporation generator 130. In embodiments, the electroporation device 60 is configured to deliver electric field energy to target tissue in the patient's heart 30 to create tissue apoptosis, rendering the tissue incapable of conducting electrical signals. Also, as will be described in greater detail below, the electroporation device 60 is configured to generate, based on models of electric fields, graphical representations of the electric fields that can be produced using the electroporation catheter 105 and to overlay, on the display 92, the graphical representations of the electric fields on an anatomical map of the patient's heart to aid a user in planning ablation (e.g., planning ablation before and during an ablation procedure) by irreversible electroporation using the electroporation catheter 105.

In embodiments, the electroporation device 60 is configured to generate the graphical representations of the electric fields based on characteristics of the electroporation catheter 105 and the position of the electroporation catheter 105 in the patient 20, such as in the heart 30 of the patient 20. In embodiments, the electroporation device 60 is configured to generate the graphical representations of the electric fields based on characteristics of the electroporation catheter 105 and the position of the electroporation catheter 105 in the patient 20, such as in the heart 30 of the patient 20, and the characteristics of the tissue surrounding the catheter 105, such as measured impedances of the tissue.

The controller 90 is configured to control functional aspects of the electroporation device 60. In embodiments, the controller 90 is configured to control the electroporation generator 130 to generate electrical pulses, for example, the magnitude of the electrical pulses, the timing and duration of electrics pulses. In embodiments, the electroporation generator 130 is operable as a pulse generator for generating and supplying pulse sequences to the electroporation catheter 105.

In embodiments, the introducer sheath 110 is operable to provide a delivery conduit through which the electroporation catheter 105 may be deployed to the specific target sites within the patient's heart 30. It will be appreciated, however, that the introducer sheath 110 is illustrated and described herein to provide context to the overall electrophysiology system 50.

As will be appreciated by the skilled artisan, the depiction of the electrophysiology system 50 shown in FIG. 1 is intended to provide a general overview of the various components of the system 50 and is not in any way intended to imply that the disclosure is limited to any set of components or arrangement of the components. For example, the skilled artisan will readily recognize that additional hardware components, e.g., breakout boxes, workstations, and the like, can and likely will be included in the electrophysiology system 50.

In the illustrated embodiment, the electroporation catheter 105 includes a handle 105a, a shaft 105b, and an electrode assembly 150. The handle 105a is configured to be operated by a user to position the electrode assembly 150 at the desired anatomical location. The shaft 105b has a distal end 105c and generally defines a longitudinal axis of the electroporation catheter 105. As shown, the electrode assembly 150 is located at or proximate the distal end 105c of the shaft 105b. In embodiments, the electrode assembly 150 is electrically coupled to the electroporation generator 130, to receive electrical pulse sequences or pulse trains, thereby selectively generating electrical fields for ablating the target tissue by irreversible electroporation.

In embodiments, as shown in FIG. 1, the electrode assembly 150 includes one or more electrodes 152. The electrodes 152 may include ablation electrodes, and optionally, mapping electrodes. In some configurations, the mapping electrodes are configured to be used to generate, and display via the display 92, detailed three-dimensional geometric anatomical maps or representations of the cardiac chambers as well as electro-anatomical maps in which cardiac electrical activity of interest is superimposed on the geometric anatomical maps.

In certain embodiments, the electroporation catheter 105 is a catheter having an electrode assembly and a plurality of states. In embodiments, the electrode assembly has a first shape when the catheter 105 is at a first state of the plurality of states, and a second shape when the catheter 105 is at a second state of the plurality of states. The electrode assembly includes a plurality of electrodes. In some embodiments, the second shape of the electrode assembly is different from the first shape. In some embodiments, the second shape of the electrode assembly is dissimilar to the first shape. In some embodiments, the second shape of the electrode assembly is smaller than the first shape in volume. The catheter 105 may have more than two (2) states. In some embodiments, the catheter 105 has continuous states, as opposed to discrete states. In other words, the catheter can be adjusted along a continuous spectrum of states and is not limited to a discrete, finite number of states. In certain examples, the electrode assembly of the catheter 105 may have more than two (2) different shapes.

In some embodiments, the controller 90 is configured to generate a graphical user interface 95 to be rendered on the display 92. In some embodiments, the controller 90 is configured to collect and store data associated with the catheter 105 and/or therapeutic sessions in a data repository (e.g., file, database, etc.). In some examples, the controller 90 is configured to generate a first graphical representation of first electric fields generated by the plurality of the electrodes based on a first model of electric fields, a second graphical representation of second electric fields generated by the plurality of the electrodes based on a second model of electric fields, and overlay the first and the second graphical representations of the electric fields on an anatomical map of a patient at a catheter location on a graphical user interface rendered on the display 92. The first graphical representation is generated when the catheter is at the first state and deployed proximate to a target location. The second graphical representation is generated when the catheter is at the second state and deployed proximate to a target location.

In some examples, the controller 90 is configured to determine electric fields based on known shapes of the electrode assembly of the catheter 105 at different states (e.g., two or more states, continuous states) and generate corresponding graphical representations of electric fields. In certain examples, the controller 90 is configured to determine electric fields based on known relative electrode locations of the catheter 105 at different states (e.g., two or more states, continuous states) and generate corresponding graphical representations of electric fields.

In some embodiments, the controller 90 is further configured to generate an indication of a difference between the first graphical representation of the first electric fields and the second graphical representation of the second electric fields. In some instances, the first graphical representation of the first electric fields includes one or more first areas, and the field strength of the first electric fields within the one or more first areas is greater than a predefined threshold in magnitude. In some instances, the second graphical representation of the second electric fields includes one or more second areas, and the field strength of the second electric fields within the one or more second area is greater than the predefined threshold in magnitude. In some embodiments, the first graphical representation includes a representation of the catheter 105.

In some embodiments, as will be explained in more details below, the controller 90 may be configured to generate a software widget including a representation the catheter 105 and an indication of one or more therapeutic sessions of electroporation ablation conducted by the catheter 105, and presenting the software widget in a graphical user interface. In embodiments, the software widget includes an indication identifying a therapeutic session of the one or more therapeutic sessions. In embodiments, the software widget includes representations of multiple therapeutic sessions where the catheter 105 is at various rotation angles, at various locations, and/or at various states with different shapes (e.g., a basket shape, a flower shape, etc.) In one example, for an ablation treatment, the catheter is deployed to conduct eight (8) therapeutic sessions, including being arranged in four (4) different rotation angles, and at each angle, arranged in two different shapes. In some embodiments, the software widget includes a cross-sectional schematic view of the catheter 105. The controller 90 may be further configured to generate a graphical representation of the electric fields of the plurality of electrodes based on a model of electric fields, and display the graphical representation of the electric fields of the plurality of electrodes in the software widget.

In some instances, the representation of the catheter 105 includes a first representation of the catheter at a first time and a second representation of the catheter of the second time. The first representation and the second representation may show a difference between the catheter at the first time and the catheter at the second time. The differences shown include at least one of a difference in shape, a difference in a rotating angle, a difference in electric field strength, and a difference in location. In some embodiments, the software widget includes a field indication representing electric fields.

In some embodiments, the software widget includes an alignment indicator representing alignment information of the catheter 105. In some examples, the alignment indicator represents axial relation between the catheter 105 at the first time and the catheter 105 at the second time, for example, a change in orientation of the catheter 105 during one therapeutic session from a prior therapeutic session. In certain examples, the alignment indicator represents axial relation between the catheter and a target ablation area of the electroporation ablation treatment.

In some embodiments, the one or more mapping electrodes on the electroporation catheter 105 can measure electrical signals and generate output signals that can be processed by the controller 90 to generate an electro-anatomical map, also referred to as an anatomical map. In some instances, electro-anatomical maps are generated before ablation for determining the electrical activity of the cardiac tissue within a chamber of interest. In some instances, electro-anatomical maps are generated after ablation in verifying the desired change in electrical activity of the ablated tissue and the chamber as a whole. The mapping electrodes may also be used to determine the position of the catheter 105 in three-dimensional space within the body. For example, when the operator moves the distal end of the catheter 105 within a cardiac chamber of interest, the boundaries of catheter movement can be used by the controller 90, which may include or couple to a mapping and navigation system, to form the anatomical map of the chamber. The chamber anatomical map may be used to facilitate navigation of the catheter 105 without the use of ionizing radiation such as with fluoroscopy, and for tagging locations of ablations as they are completed in order to guide spacing of ablations and aid the operator in fully ablating the anatomy of interest.

According to embodiments, various components (e.g., the controller 90) of the electrophysiological system 50 may be implemented on one or more computing devices. A computing device may include any type of computing device suitable for implementing embodiments of the disclosure. Examples of computing devices include specialized computing devices or general-purpose computing devices such as workstations, servers, laptops, portable devices, desktop, tablet computers, hand-held devices, general-purpose graphics processing units (GPGPUs), and the like, all of which are contemplated within the scope of FIG. 1 with reference to various components of the system 50.

In some embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in some embodiments, the computing device may include a number of processors, a number of memory components, a number of I/O ports, a number of I/O components, and/or a number of power supplies. Additionally, any number of these components, or combinations thereof, may be distributed and/or duplicated across a number of computing devices.

In some embodiments, the system 50 includes one or more memories (not illustrated). The one or more memories includes computer-readable media in the form of volatile and/or nonvolatile memory, transitory and/or non-transitory storage media and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In some embodiments, the one or more memories store computer-executable instructions for causing a processor (e.g., the controller 90) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Program components may be programmed using any number of different programming environments, including various languages, development kits, frameworks, and/or the like. Some or all of the functionality contemplated herein may also, or alternatively, be implemented in hardware and/or firmware.

In some embodiments, the memory may include a data repository may be implemented using any one of the configurations described below. A data repository may include random access memories, flat files, XML files, and/or one or more database management systems (DBMS) executing on one or more database servers or a data center. A database management system may be a relational (RDBMS), hierarchical (HDBMS), multidimensional (MDBMS), object oriented (ODBMS or OODBMS) or object relational (ORDBMS) database management system, and the like. The data repository may be, for example, a single relational database. In some cases, the data repository may include a plurality of databases that can exchange and aggregate data by data integration process or software application. In an exemplary embodiment, at least part of the data repository may be hosted in a cloud data center. In some cases, a data repository may be hosted on a single computer, a server, a storage device, a cloud server, or the like. In some other cases, a data repository may be hosted on a series of networked computers, servers, or devices. In some cases, a data repository may be hosted on tiers of data storage devices including local, regional, and central.

Various components of the system 50 can communicate via or be coupled to via a communication interface, for example, a wired or wireless interface. The communication interface includes, but not limited to, any wired or wireless short-range and long-range communication interfaces. The wired interface can use cables, umbilicals, and the like. The short-range communication interfaces may be, for example, local area network (LAN), interfaces conforming known communications standard, such as Bluetooth^{®} standard, IEEE 802 standards (e.g., IEEE 802.11), a ZigBee^{®} or similar specification, such as those based on the IEEE 802.15.4 standard, or other public or proprietary wireless protocol. The long-range communication interfaces may be, for example, wide area network (WAN), cellular network interfaces, satellite communication interfaces, etc. The communication interface may be either within a private computer network, such as intranet, or on a public computer network, such as the internet.

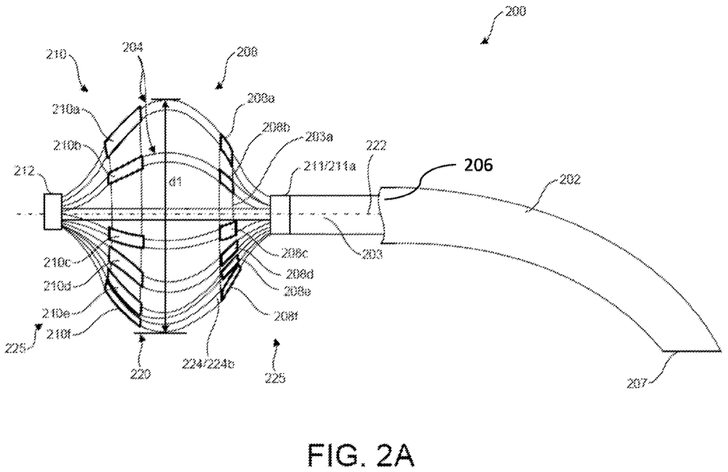

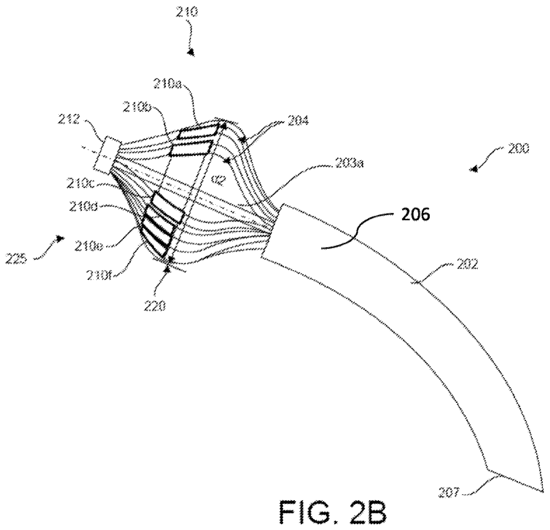

FIGS. 2A-2B are schematic views illustrating electroporation ablation catheter 200 that can be used for electroporation ablation, including ablation by irreversible electroporation, in accordance with embodiments of the subject matter of the disclosure. FIG. 2A is a diagram illustrating the catheter 200 in a first state; FIG. 2B is a diagram illustrating the catheter 200 in a second state. The catheter 200 may have two or more states, where the states are either configurable or controllable by a user or automatically configurable by an electroporation system during treatment. The catheter 200 includes a catheter shaft 202 and a plurality of catheter splines 204 connected to the catheter shaft 202 at the distal end 206 of the catheter shaft 202. The catheter 200 may further include an inner shaft 203 disposed within the catheter shaft 202 and extending distally from a distal end 206 of the catheter shaft 202. As will be appreciated, the catheter shaft 202 is coupled, at its proximal end, to a handle assembly (not shown) configured to be manipulated by a user during an electroporation ablation procedure. As further shown, the catheter 200 includes an electrode assembly 220 at a distal end extending from the distal end 206 of the catheter shaft 202.

In embodiments, the electrode assembly 220 includes a plurality of energy-delivering electrodes 225, where the electrode assembly 220 is configured to be selectively operable in a first state and a second state. In some cases, in the first state the electrode assembly 220 is configured to deliver ablative energy to form circumferential ablation lesion having a certain diameter.

In some embodiments, the electrode assembly 220 includes an inner shaft 203, where the inner shaft 203 is adapted to be extended from and retracted into the catheter shaft 202. In some cases, the electrode assembly 220 includes a plurality of splines 204 connected to the inner shaft 203 at a distal end 211 of the inner shaft 203. In some cases, the electrode assembly 220 further includes a center shaft 203a having a proximal end 211a (overlapped with the distal end 211 of the inner shaft 203) and a distal end 212. In some cases, the plurality of splines 204 are connected to the distal end 212 of the center shaft 203a. In embodiments, the electrodes 225 includes a plurality of first electrodes 208 and a plurality of second electrodes 210 disposed on the plurality of splines 204. In one example, the plurality of second electrodes 210 are disposed close to the distal end 212 of the center shaft 203a and the plurality of first electrodes 208 are disposed close to the proximal end 211a of the center shaft 203a.

In some cases, when operating in the first state, the inner shaft 203 and the center shaft 203a are extended from the catheter shaft 202, for example, as illustrated in FIG. 2A. In some cases, in the first state, both the plurality of first electrodes 208 and the plurality of second electrodes 210 are activated selectively energized to form relatively large diameter for circumferential ablation lesions, for example, used in a pulmonary vein isolation (PVI) procedure.

In some embodiments, when operating in the second state, the inner shaft 203 and the center shaft 203a are at least partially retracted into the catheter shaft 202 such that all or a part of the plurality of first electrodes 208 are retracted into the catheter shaft 202, for example, as illustrated in FIG. 2B. In some cases, in the second state, the plurality of first electrodes 208 are deactivated (e.g., by electrically disconnecting the first electrodes 208 from any pulse generator circuitry) and the plurality of second electrodes 210 are activated and used to create focal ablation lesions via electroporation.

The ablation catheter 200 has a longitudinal axis 222. As used herein, a longitudinal axis refers to a line passing through the centroid of the cross sections of an object. In embodiments, the plurality of splines 204 forms a cavity 224. The plurality of splines 204 forms a cavity 224a in the first state and forms a cavity 224b in the second state. In embodiments, the cavity 224a is larger than the cavity 224b in volume. In some embodiments, in the first state, the largest cross-sectional area generally perpendicular to the longitudinal axis 222 of the cavity 224a has a diameter d1. In some embodiments, in the second state, the largest cross-sectional area generally perpendicular to the longitudinal axis 222 of the cavity 224b has a diameter d2. In some cases, the diameter d1 is larger than the diameter d2.

In some examples, the diameter d1 is in the range of twenty (20) millimeters and thirty-five (35) millimeters. In certain examples, the diameter d1 is in the range of ten (10) millimeters and twenty-five (25) millimeters. In some examples, the diameter d2 is in the range of five (5) millimeters and sixteen (16) millimeters. In some examples, the diameter d2 is in the range of five (5) millimeters and sixteen (16) millimeters. In one example, the diameter d1 is greater than the diameter d2 by 30% to 100%. In one example, the diameter d1 is greater than the diameter d2 by at least 30%. In one example, the diameter d1 is greater than the diameter d2 by at least 20%. In one example, the diameter d1 is greater than the diameter d2 by at least 100% (i.e., at least two times of the diameter d2). In one example, the diameter d1 is greater than the diameter d2 by at least 150% (i.e., at least two and a half times of the diameter d2).

In some cases, the first group of electrodes 208 disposed at or proximate the circumference of the plurality of splines 204 and the second group of electrodes 210 disposed proximate to the distal end 212 of the catheter 200. In some cases, the first group of electrodes 208 are referred to as proximal electrodes, and the second group of electrodes 210 are referred to as distal electrodes, where the distal electrodes 210 are disposed closer to the distal end 212 of the electroporation ablation catheter 200 than the proximal electrodes 208. In some implementations, the electrodes 225 can include a thin film of an electro-conductive or optical ink. The ink can be polymer-based. The ink may additionally comprise materials such as carbon and/or graphite in combination with conductive materials. The electrode can include a biocompatible, low resistance metal such as silver, silver flake, gold, and platinum which are additionally radiopaque.

Each of the electrodes in the first group of electrodes 208 and each of the electrodes in the second group of electrodes 210 is configured to conduct electricity and to be operably connected to a controller (e.g., the controller 90 in FIG. 1) and an ablative energy generator (e.g., the electroporation generator 130 of FIG. 1). In embodiments, one or more of the electrodes in the first group of electrodes 208 and the second group of electrodes 210 includes flex circuits. In some cases, the plurality of first electrodes 208 are individually controllable. In some cases, the plurality of second electrodes are individually controllable. In some cases, all or a part of the plurality of first electrodes 208 are deactivated in the second state. In some cases, a part of the plurality of second electrodes 210 are deactivated in the second state.

Electrodes in the first group of electrodes 208 are spaced apart from electrodes in the second group of electrodes 210. The first group of electrodes 208 includes electrodes 208a-208f and the second group of electrodes 210 includes electrodes 210a-210f. Also, electrodes in the first group of electrodes 208, such as electrodes 208a-208f, are spaced apart from one another and electrodes in the second of electrodes 210, such as electrodes 210a-210f, are spaced apart from one another.

The spatial relationships and orientation of the electrodes in the first group of electrodes 208 and the spatial relationships and orientation of the electrodes in the second group of electrodes 210 in relation to other electrodes on the same catheter 200 is known or can be determined. In embodiments, the spatial relationships and orientation of the electrodes in the first group of electrodes 208 and the spatial relationships and orientation of the electrodes in the second group of electrodes 210 in relation to other electrodes on the same catheter 200 is constant, once the catheter is deployed.

As to electric fields, in embodiments, each of the electrodes in the first group of electrodes 208 and each of the electrodes in the second group of electrodes 210 can be selected to be an anode or a cathode, such that electric fields can be set up between any two or more of the electrodes in the first and second groups of electrodes 208 and 210. Also, in embodiments, each of the electrodes in the first group of electrodes 208 and each of the electrodes in the second group of electrodes 210 can be selected to be a biphasic pole, such that the electrodes switch or take turns between being an anode and a cathode. Also, in embodiments, groups of the electrodes in the first group of electrodes 208 and groups of the electrodes in the second group of electrodes 210 can be selected to be an anode or a cathode or a biphasic pole, such that electric fields can be set up between any two or more groups of the electrodes in the first and second groups of electrodes 208 and 210.

In embodiments, electrodes in the first group of electrodes 208 and the second group of electrodes 210 can be selected to be biphasic pole electrodes, such that during a pulse train including a biphasic pulse train, the selected electrodes switch or take turns between being an anode and a cathode, and the electrodes are not relegated to monophasic delivery where one is always an anode and another is always a cathode. In some cases, the electrodes in the first and second group of electrodes 208 and 210 can form electric fields with electrode(s) of another catheter. In such cases, the electrodes in the first and second group of electrodes 208 and 210 can be anodes of the fields, or cathodes of the fields.

Further, as described herein, the electrodes are selected to be one of an anode and a cathode, however, it is to be understood without stating it that throughout the present disclosure the electrodes can be selected to be biphasic poles, such that they switch or take turns between being anodes and cathodes. In some cases, one or more of the electrodes in the first group of electrodes 208 are selected to be cathodes and one or more of the electrodes in the second group of electrodes 210 are selected to be anodes. In embodiments, one or more of the electrodes in the first group of electrodes 208 can be selected as a cathode and another one or more of the electrodes in the first group of electrodes 208 can be selected as an anode. In addition, in embodiments, one or more of the electrodes in the second group of electrodes 210 can be selected as a cathode and another one or more of the electrodes in the second group of electrodes 210 can be selected as an anode.

In some instances, the first group of electrodes 208 is disposed proximal the maximum circumference (d1) of the catheter splines 204 and the second group of electrodes 210 disposed distal the maximum circumference of the catheter splines 204. In some embodiments, additional electrodes (i.e. mapping electrodes) may be added to each of the plurality of splines 204.

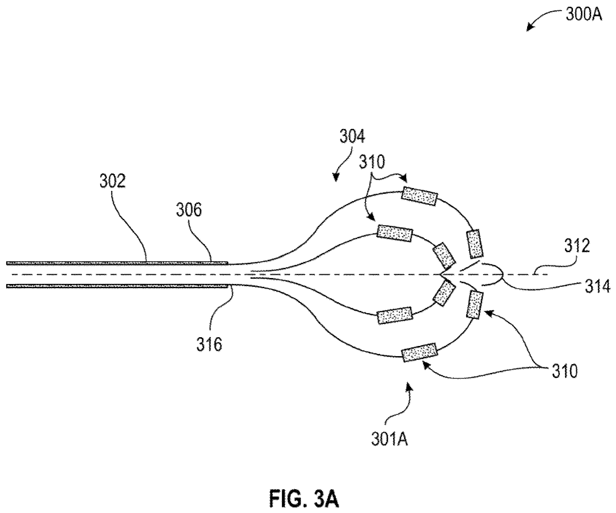

FIGS. 3A-3C are schematic views illustrating ablation catheter 300 that can be used for electroporation ablation, including ablation by irreversible electroporation, in accordance with embodiments of the subject matter of the disclosure.

FIG. 3A shows a catheter 300A in a first state, or referred to as a first operation mode. In some embodiments, the catheter 300A includes an electrode assembly 301A. The electrode assembly 301A has a first shape, or referred to as a basket shape, in FIG. 3A. The catheter 300 includes a catheter shaft 302. The electrode assembly includes a plurality of splines 304 connected to the catheter shaft 302 at the distal end 306 of the catheter shaft 302. The catheter splines 304 includes a plurality of electrodes 310 disposed on the catheter splines 204. Each of the electrodes in the plurality of electrodes 310 is configured to conduct electricity and to be operably connected to the electroporation generator (e.g., the electroporation generator 130 in FIG. 1). In embodiments, one or more of the electrodes in the plurality of electrodes 310 includes metal.

The electrode assembly 301A has a proximal end 316 close to the distal end 306 of the catheter shaft 302 and a distal end 314 further away from the distal end 306 of the catheter shaft 302. As shown, the catheter shaft 302 defines a longitudinal axis 312, and the plurality of splines 304 are arranged in a curved shape between the distal end 314 and the proximal end 160. In embodiments, each spline 304 of the electrode assembly 301 in the first state is arranged as a curve with no turning point. In some examples, each spline 304 has a degree of curvature less than a predetermined degree. For example, each spline 304 has a degree of curvature less than less than 45°.

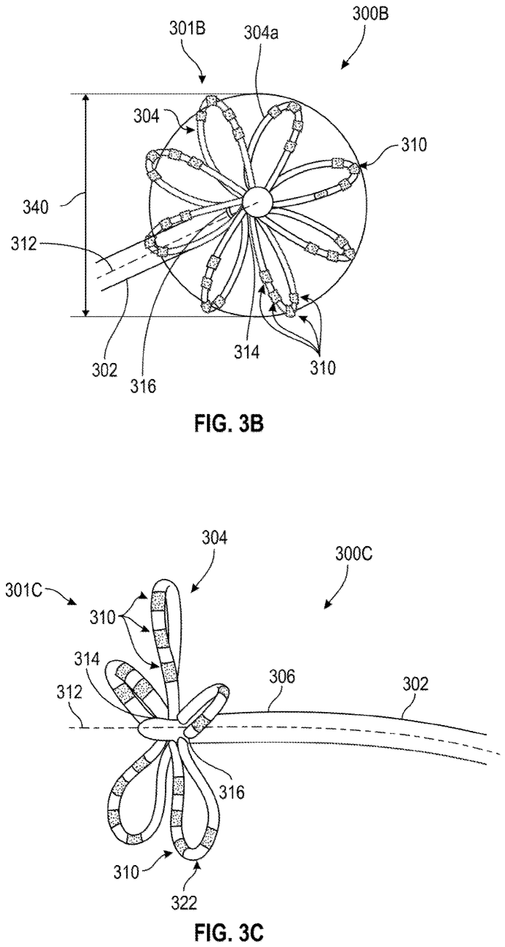

FIG. 3B show a catheter 300B and 300C in a second state from an end view or referred to as a second operation mode; and FIG. 3C show a catheter 300C in a second state from a side view. In embodiments, each of the plurality of splines 304 includes one or more electrodes 310 disposed thereon. For example, as shown, spline 304a includes 4 electrodes. In some embodiments, each of the plurality of splines 304 may include more than 4 electrodes. In some embodiments, each of the plurality of splines 304 may include fewer than 4 electrodes. As may be understood by a skilled artisan, the number of electrodes on each spline may be adjusted, including the spacing between each electrode. The catheter shaft may further include a cap 324. In embodiments, the cap 324 is atraumatic to reduce trauma to tissue

Each of the plurality of splines 304 as shown has similar size, shape, and spacing between adjacent electrodes 310 on a spline 304. In other embodiments, the size, shape, and spacing between adjacent electrodes 310 on a spline 304 may differ. In some embodiments, the thickness and length of each of the plurality of splines 304 may vary based on the number of electrodes and spacing between each electrode on the splines 304. The splines 304 may be made from similar of different materials, and may vary in thickness or length.

As shown, each of the plurality of splines 304 are arranged in a petal-like curve 322 in the second state, where the distal end 314 of the electrode assembly 301 is adjacent the proximal end 316 of the electrode assembly 301. Each of the plurality of splines 304 may pass through the distal end 306 of the catheter shaft 302 and be tethered to the catheter shaft 302 within a catheter shaft lumen. The distal end of each of the plurality of splines 304 may be tethered to the cap 324 of the catheter 300. In some embodiments, the one or more curve 322 are electrically isolated. As shown, the petal-like curve 322 includes a turning point.

In some embodiments, the catheter 300B includes an electrode assembly 301B arranged in a second shape, or referred to as a flower shape, as shown in FIG. 3B. In some embodiments, the catheter 300C includes an electrode assembly 301C arranged in the second shape as shown in FIG. 3C. As shown, each of the plurality of splines 304 may include a flexible curvature so as to rotate, or twist and bend and form the petal-shaped curve 322. The minimum radius of curvature of a spline in the petal-shaped configuration may be in the range of about 7 mm to about 25 mm. For example, the splines 304 may form an electrode assembly 301 at a distal portion of the catheter 300 and be configured to transform between a first shape where the set of splines are arranged generally parallel to the longitudinal axis of the catheter 300, and a second shape where the set of splines rotate around, or twist and bend, and generally bias away from the longitudinal axis of the catheter 300. In the first shape, each spline of the set of splines 304 may lie in one plane with the longitudinal axis 312. In the second shape, each spline of the set of splines 304 may bias away from the longitudinal axis 312 to form a petal-like curve 322 arranged generally perpendicular to the longitudinal axis 312. In this manner, the set of splines 304 twist and bend and bias away from the longitudinal axis 312 of the catheter 300, thus allowing the splines 304 to more easily conform to the geometry of an endocardial space, and particularly adjacent to the opening of a pulmonary ostium. The second shape may, for example, resemble the shape of a flower, from an end view as shown in FIG. 3B. In some embodiments, each spline in the set of splines in the second configuration may twist and bend to form a petal-like curve that, when viewed from front, displays an angle of curvature between the proximal and distal ends of the curve of proximate to 180 degrees.

The set of splines may further be configured to transform from a second shape to a third shape where the set of splines 304 may be impressed (e.g., in contact with) against target tissue such as tissue surrounding a pulmonary vein ostium. The plurality of splines 304 may form a shape generally parallel to a longitudinal axis 312 of the catheter shaft 302 when undeployed, be wound (e.g., helically, twisted) about an axis (not shown) parallel to the longitudinal axis 312 when fully deployed, and form any intermediate shape (such as a cage or barrel) in-between the various the shapes.

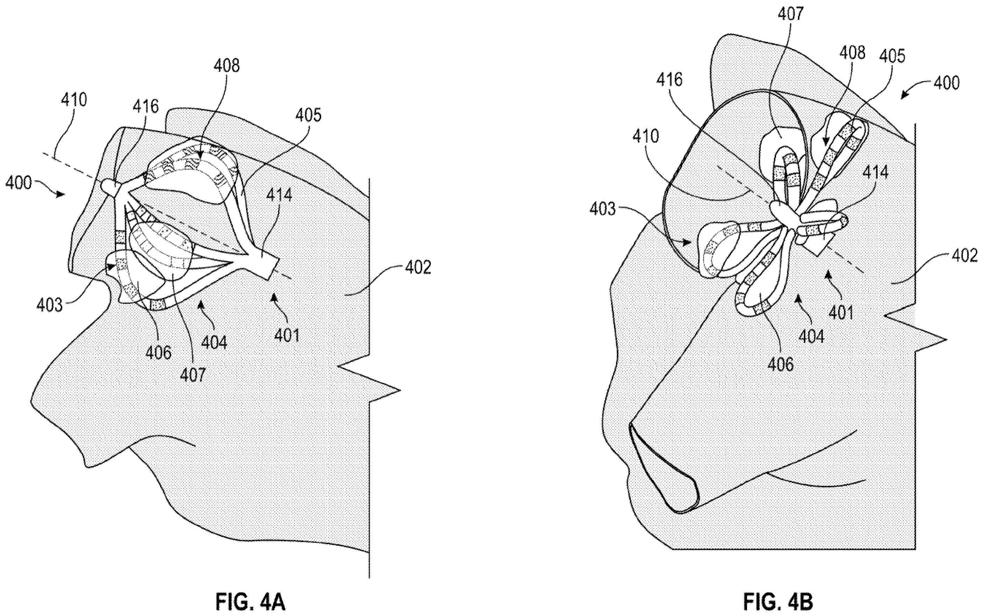

FIGS. 4A-4B are diagrams illustrating examples of graphical user interface 400, in accordance with embodiments of the subject matter of the disclosure. As discussed above, the electroporation catheter system (e.g., the electroporation device 60 in FIG. 1) including a controller (e.g., the controller 90 in FIG. 1) generates, based on models of electric fields, graphical representations 403 of the electric fields that can be produced using the electroporation catheter and to overlay the graphical representations 403 of the electric fields on an anatomical map 402 of the patient's heart. This graphical representation may aid a user in planning ablation by irreversible electroporation and/or give real-time visual feedback on the progress of the ablation for the user to adjust the ablation plans accordingly. In embodiments, the controller (e.g., the controller 90 in FIG. 1) is configured to display these and other graphical representations in the overlay of the graphical representation of the electric field on the anatomical map on a display (e.g., the display 92 in FIG. 1).

In some embodiments, the controller (e.g., the controller 90 in FIG. 1) is configured to display only a three-dimensional surface without thickness in the overlay of the graphical representation of the electric field on the anatomical map. In some embodiments (not shown), the graphical representation 403 may include different colors to represent different strengths of the projected electric field. In some embodiments (not shown), the graphical representation 403 may include a gradient or vector field to represent different strengths of the projected electric field.

As shown, a catheter 405 including an electrode assembly 401 is deployed within a vein of a patient's heart. The graphical user interface includes a graphical representation 406 of electric fields that have an ablation impact to the surrounding tissues on the anatomical map 402 of a patient's vein. Axis 410 is the projected axis of the catheter 405 to be aligned with an axis of the vein (not shown). In some examples, the graphical representation 406 includes areas 407, where field strength of electric fields in the areas 407 is greater than a predetermined threshold. In some examples, the graphical representation 406 includes areas 407, where field strength of electric fields in the areas is greater than an electroporation threshold (e.g., 250 V/cm). In certain examples, the graphical representation 406 includes areas 407, where field strength of electric fields in the areas is greater than an irreversible electroporation threshold (e.g., 400 V/cm). Since the graphical user interface 400 includes a representation of the catheter 405, the longitudinal axis 410, the anatomical map 402, and the predicted electric field as a function of the deployed shape of the catheter 405, it enables the user to advance or navigate the catheter 405 during treatment sessions without the need for fluoroscopy.

The electrode assembly includes a plurality of splines 404 including one or more electrodes 408 disposed thereon. The one or more electrodes 408 may include ablation electrodes and/or mapping electrodes. In some examples, the one or more electrodes may be disposed at other components of the catheter 405, such as the end cap at the distal end 416 and the catheter shaft (not shown).

In some embodiments, FIGs. 4A and 4B are updated in real time showing catheter positions and electric fields generated by the catheter 405. In certain embodiments, the graphical representation 406 of electric fields includes one or more indicators including, for example, color representing field strength, tissue contacts, and locations. In some examples, the graphical representation 406 may be in a different color from the color of the previous graphical representation 406 if the catheter 405 has moved to a new location at the present therapeutic session from a previous location of the previous therapeutic session associated with the previous graphical representation. In certain examples, the graphical representation 406 may be in a different color from the color of the previous graphical representation 406 if the catheter 405 has rotated from the previous therapeutic session associated with the previous graphical representation. In some examples, a color is a grayscale color. In certain embodiments, FIGs. 4A and 4B are presented side-by-side to show changes in therapeutic sessions. In some embodiments, the electric fields of the catheter 405 are generated based on known relative locations of electrodes in the catheter.

FIG. 4A shows the catheter 405 in a first state where the plurality of splines 404 are arranged in curves between the proximal end 414 of the electrode assembly 401 and the distal end 416 of the electrode assembly 401, as a first shape (e.g., a basket shape). FIG. 4B shows the catheter 405 in a second state where the plurality of splines 404 are arranged in petal-like curves, as a second shape (e.g., a flower shape). In some embodiments, the splines 404 are electrically isolated. When the electrode assembly 401 is in a first shape, the splines on the electrode assembly 401 may be arranged generally parallel to the longitudinal axis of the catheter 405. In the first shape, each spline may lie in one plane with the longitudinal axis 410.

In some embodiments, as shown in FIGS. 4A and 4B, the first shape of electrode assembly 401 when the catheter 405 is in the first state is dissimilar to the second shape of the electrode assembly 401 when the catheter 405 is in the second state. In other embodiments, shown in FIGs. 2A-2B, the shape of the electrode assembly in the first state is different from the shape of the splines in the second state in volume.

As discussed above, a controller (e.g., the controller 90 in FIG. 1) is configured to control an electroporation generator (e.g., the electroporation generator 130 in FIG. 1) to generate electrical pulses and deliver therapy to targeted areas within a patient's cardiac chamber. In some embodiments, the controller may be configured to generate a first graphical representation of the electric fields of the plurality of the electrodes in the first state based on a first model of electric fields, a second graphical representation of the electric fields of the plurality of the electrodes in the second state based on a second model of electric fields, and overlay the first and the second graphical representations of the electric fields on an anatomical map of a patient at a target location, where the catheter 405 is deployed.

When the electrode assembly 401 is in a second shape, the splines on the electrode assembly 401 rotate around, or twist and bend, and generally bias away from the longitudinal axis of the catheter 405. In the second shape, each spline may bias away from the longitudinal axis 410 to form a petal-like curve arranged generally perpendicular to the longitudinal axis 410. In this manner, the set of splines 304 twist and bend and bias away from the longitudinal axis 410, thus allowing the splines to more easily conform to the geometry of an endocardial space, and particularly adjacent to the opening of a pulmonary ostium. The second shape may, for example, resemble the shape of a flower, from an end view as shown in FIG. 4B. In some embodiments, the each spline in the set of splines in the second configuration may twist and bend to form a petal-like curve that, when viewed from front, displays an angle of curvature between the proximal and distal ends of the curve of proximate to 180 degrees.

In addition to what is shown in FIGs. 4A and 4B, the set of splines may further be configured to transform from a second shape to a third shape where the splines may be impressed (e.g., in contact with) against target tissue such as tissue surrounding a pulmonary vein ostium. The splines may form a shape generally parallel to a longitudinal axis 410 when undeployed, be wound (e.g., helically, twisted) about an axis (not shown) parallel to the longitudinal axis 410 when fully deployed, and form any intermediate shape (such as a cage or barrel) in-between the various the shapes.

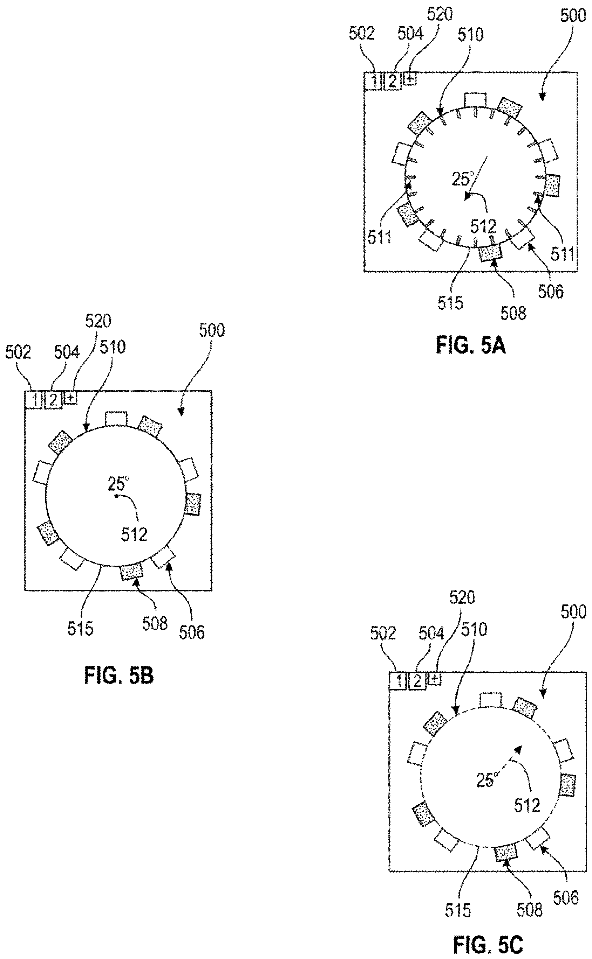

FIGS. 5A-5C illustrate a software widget 500 to facilitate planning and/or conducting ablation treatment, in accordance with certain embodiments of the present disclosure. In implementations, the software widget 500 is implemented by a set of software instructions executed by one or more processors. As shown, the numbers 502, 504 represent the number of ablation therapeutic sessions (e.g., session 1, session 2). 502 shows a previous therapy session, and 504 shows the current therapy session the user is on. In some examples, the plurality of squares 506, 508 located on the outer side of the circle 515 represent locations of electrodes of the catheter in a cross-sectional view. In certain examples, the plurality of squares 506, 508 located on the outer side of the circle represent locations of the splines on the electrode assembly as viewed from a cross-sectional view. 506 represents the locations of the splines during the first (past) session, and 508 represent the locations of the splines during the second (current) session. In some instances, as shown, the catheter is rotated 25 degrees between the first and second session. In other instances, not shown, the angle of rotation may be larger or smaller than 25 degrees.

In embodiments, the catheter representation 510 indicate relative position of the electrode assembly in between each session. As shown in FIG. 5A, the catheter representation 510 with a plurality of teeth 511 located on the inner side of the circle 515 indicates that the electrode assembly is in a more distal position along a patient's vein compared to a previous application/session. As shown in FIG. 5B, the circle 5155 with a solid line indicates that the position of the electrode assembly remains substantially the same in a patient's vein compared to a previous application/session. As shown in FIG. 5C, the circle 515 with a dashed line indicates that the position of the electrode assembly is in a more antral or proximal position along a patient's vein or cardiac chamber compared to a previous application/session.

In embodiments, the software widget 500 includes an alignment indicator 512. In some examples, as shown in FIGS. 5A and 5C, the alignment indicator 512 is an arrow or dash-line arrow indicating misalignment of a projected axis of a catheter (e.g., an axis defined by a catheter shaft of the catheter) with an axis of the target ablation area in a patient's vein or cardiac chamber. The alignment indicator 512 can have various shapes and/or color. The alignment indicator 512, as shown in FIG. 5B, is a dot indicating that the projected axis of the catheter shaft is substantially aligned with the axis of the target ablation area in a patient's vein or cardiac chamber.

In some embodiments, a controller (e.g., the controller 90 in FIG. 1) may be configured to generate the software widget 500 including a representation of the catheter and an indication of one or more therapeutic sessions of electroporation ablation (not shown in FIGS. 5A-5C) and display the software widget 500. In embodiments, the software widget includes an indication identifying a therapeutic session of the one or more therapeutic sessions. In some embodiments, the software widget includes a cross-sectional view of the catheter (e.g., the catheter 105 in FIG. 1). The controller may be further configured to generate a graphical representation of the electric fields of the plurality of electrodes based on a model of electric fields, and display the graphical representation of the electric fields of the plurality of electrodes.

In embodiments, the software widget 500 includes an alignment indicator 512 representing axial relation between the catheter and a target ablation area of the electroporation ablation treatment. In some instances, the representation the catheter 105 includes a first representation of the catheter at a first time and a second representation of the catheter at a second time. The first representation and the second representation may show a difference between the catheter at the first time and the catheter at the second time. The differences shown include at least one of a difference in shape, a difference in a rotating angle, and a difference in location. In some embodiments, the software widget further includes a field indication representing electric fields.

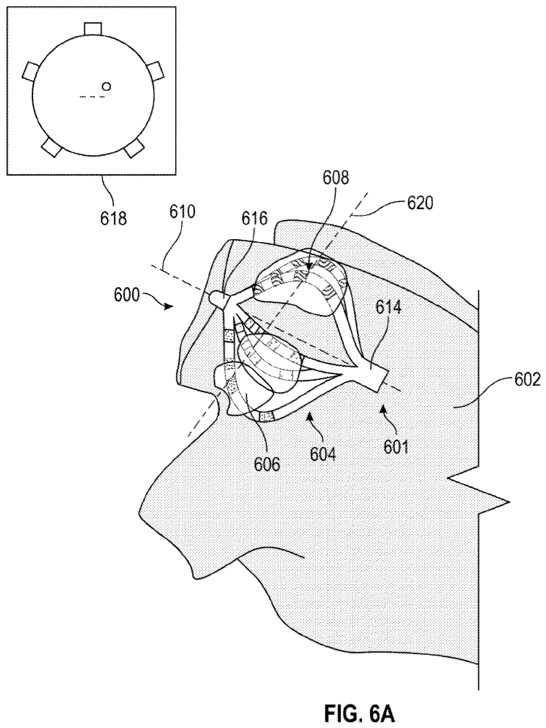

FIGS. 6A-6B are diagrams illustrating a software widget and graphical representation of electric fields displayed side-by-side, in accordance with embodiments of the subject matter of the disclosure.

As shown, a catheter 600 including an electrode assembly 601 is deployed within a patient's cardiac chamber. The display (e.g., the display 92 in FIG. 1) shows a graphical representation 606 of electric field that has an ablation impact to the surrounding tissues on the anatomical map 602 of a patient's cardiac chamber. Axis 610 is the projected axis of the catheter 600 to be aligned with an axis of the target ablation area (not shown).

The electrode assembly includes a plurality of splines 604 including one or more electrodes 608 disposed on each of the plurality of splines 604. The one or more electrodes 608 may include ablation electrodes and/or mapping electrodes. Each of the plurality of splines 604 may include additional electrodes other than the one or more electrodes 608.

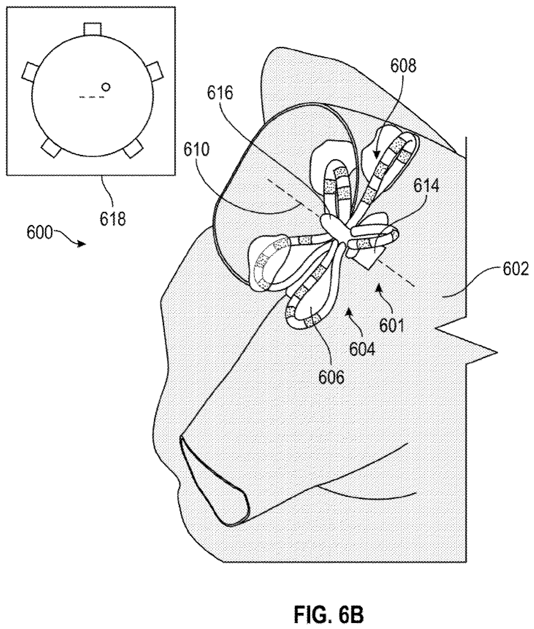

FIG. 6A shows the catheter 600 in a first state where plurality of splines 604 are arranged in a basket shape between a distal end 614 of the electrode assembly 601 and a proximal end 616 of the electrode assembly 601. FIG. 6B shows the catheter 600 in a second state where the plurality of splines 604 are arranged in a flower shape with pedal-like loops. In some embodiments, the loops are electrically isolated.

In some embodiments, as shown, the curved shape of the first state is dissimilar to the loop shape of the second state. In other embodiments, shown previously in FIG 2A-2B, the shape of the splines in the first state is merely different from the shape of the splines in the second state.

As discussed above, a controller (e.g., the controller 90 in FIG. 1) is configured to control the electroporation generator (e.g., the electroporation generator 130 in FIG. 1) to generate electrical pulses and deliver therapy to targeted areas within a patient's vein. In some embodiments, the controller may be configured to generate a first graphical representation of the electric fields of the plurality of the electrodes in the first state based on a first model of electric fields, a second graphical representation of the electric fields of the plurality of the electrodes in the second state based on a second model of electric fields, and overlay the first and the second graphical representations of the electric fields on an anatomical map of a patient at a catheter location.

In some embodiments, the controller (e.g., the controller 90 in FIG. 1) may be configured to generate the software widget 618 including a representation the catheter 600 and an indication of one or more therapeutic sessions of electroporation ablation (not shown) and display the software widget 618. The software widget 618 may include any embodiments and configurations as described herein. In embodiments, the software widget includes an indication identifying a therapeutic session of the one or more therapeutic sessions. In some embodiments, the software widget includes a cross-sectional view of the catheter 600 where the line 620 is indicating in FIG. 6A. The controller may be further configured to generate a graphical representation of the electric fields of the plurality of electrodes based on a model of electric fields, and display the graphical representation of the electric fields of the plurality of electrodes.

In embodiments, the software widget 618 includes an alignment indicator representing axial relation between the catheter and a target ablation area of the electroporation ablation treatment. In some instances, the representation the catheter 105 includes a first representation of the catheter at a first time and a second representation of the catheter at a second time. The first representation and the second representation may show a difference between the catheter at the first time and the catheter at the second time. The differences shown include at least one of a difference in shape, a difference in a rotating angle, and a difference in location. In some embodiments, the software widget further includes a field indication representing electric fields (not shown).

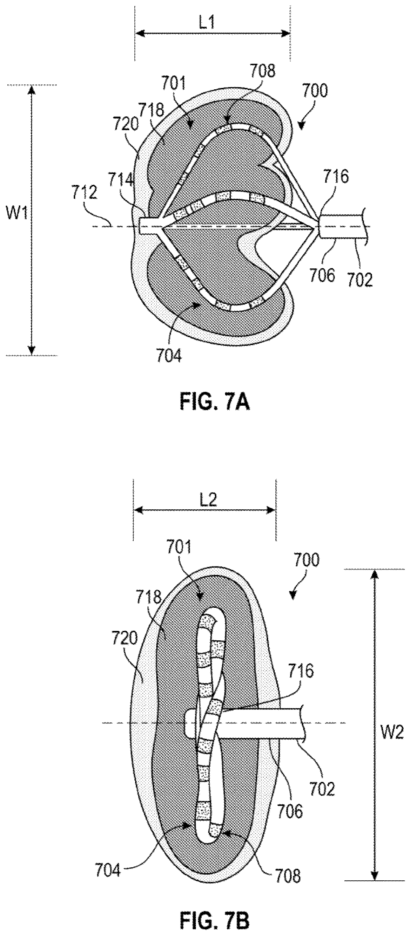

FIGS. 7A-7B illustrates example electric fields with various strength generated by a catheter including an electrode assembly. As shown, the catheter 700 includes a catheter shaft 702 and an electrode assembly 701. The electrode assembly includes a plurality of splines 704 connected to the distal end 706 of the shaft 702. Each of the plurality of splines 704 includes one or more electrodes 708. The catheter shaft 702 defines a longitudinal axis 712 extending along the length of the shaft 702.

FIG. 7A depicts the catheter 700 in a first state where the plurality of splines 704 are arranged in a curved shape between a distal end 714 and a proximal end 716 of the electrode assembly 701. As shown, the proximal end 716 of the electrode assembly 701 extends from the catheter shaft 702 of the flexible catheter 700.

FIG. 7B depicts the catheter 700 in a second state where the plurality of splines 704 are arranged in petal-like curves between a distal end 714 and a proximal end 716 of the electrode assembly 701. As shown, the distal end 714 is further apart from the proximal end 716 of the electrode assembly 701 in the first state compared to the second state. Therefore, the depth (L1) of the electric field generated in the first state is larger than the depth (L2) of the electric field generated in the second state. Similarly, the width (W1) of the electric field generated in the first state is shorter than the width (W2) of the electric field generated in the second state.

The inner electric field 718 is located closer to the electrode assembly 701 and is relatively stronger than the outer electric field 720 located further away from the electrode assembly 701. In some instances, the inner electric field 718 may be around or higher than 400 volts per centimeter. In some instances, the outer electric field 720 may be around or higher than 250 volts per centimeter but less than 400 volts per centimeter. In some embodiments, the inner electric field 718 may be strong enough to perform irreversible electroporation. In some embodiments, the outer electric field 720 may be only strong enough to perform reversible electroporation.

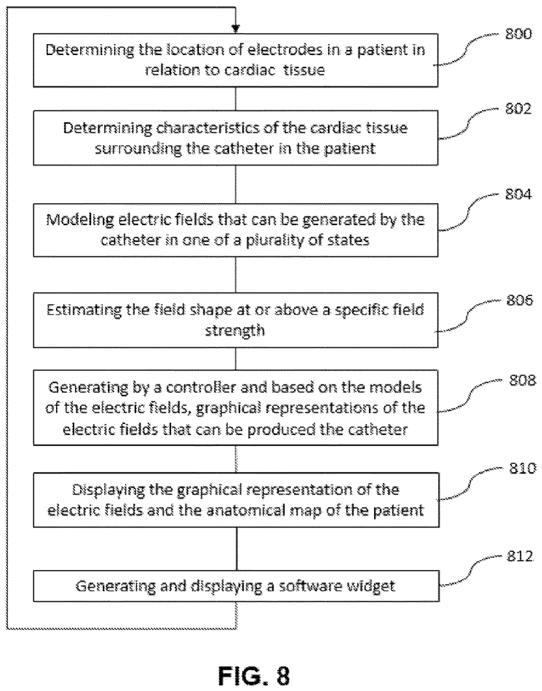

FIG. 8 is a flow chart diagram illustrating a method of planning ablation by irreversible electroporation, in accordance with embodiments of the subject matter of the disclosure. The method is described in relation to the catheters discussed previously here, however, any suitable electroporation catheter can be used in the method. Aspects of embodiments of the method may be performed, for example, by an electrophysiology system or a controller (e.g., the system 50 in FIG. 1, the controller 90 in FIG. 1). One or more steps of method are optional and/or can be modified by one or more steps of other embodiments described herein. Additionally, one or more steps of other embodiments described herein may be added to the method.

At 800, the method includes determining the location of the electrodes in the patient in relation to the cardiac tissue, after the catheter has been inserted into the patient and, at 802, the method includes determining characteristics of the cardiac tissue near or surrounding the catheter in the patient.

At 804, the method includes modeling electric fields that can be generated by the catheter in one of a plurality of states. Also, in some embodiments, the method includes selecting electrodes of the electrodes, which appear to be best suited for ablating the targeted cardiac tissue by electroporation, including ablation of targeted surface tissue and deeper tissue. In embodiments, this includes providing user inputs like voltage amplitude. In embodiments, the method includes modeling electric fields that can be generated by the electrode assembly of the catheter in different shapes. In some examples, the electrode assembly of the catheter has a shape in a first state different from the shape in the second state. In embodiments, the electrodes of the catheter have known relative positions when the catheter is in various states.

In some embodiments, at 804, the method includes generating, by a controller and based on a first model of electric fields, a first graphical representation of first electric fields produced using electrodes on a catheter at a first state, the catheter including an electrode assembly having a first shape when the catheter is at the first state. In some embodiments, at 804, the method includes generating, by a controller and based on a second model of electric fields, a second graphical representation of second electric fields produced using electrodes on a catheter at a second state, the catheter including an electrode assembly having a second shape when the catheter is at the second state, the second shape being different from the first shape. In some instances, the second shape is dissimilar to the first shape.

In some embodiments, the method may further include generating an indication of a difference between the first graphical representation of the first electric fields and the second graphical representation of the second electric fields.

At 806, the method includes estimating the field shape at a specified field strength, for example, including the surface area and depth of the cardiac tissue that will be or would be affected by an electric field. In embodiments, the method includes determining the strength of the electric field in different portions of the cardiac tissue. In some examples, the electric fields are determined with a degree of uncertainty. In embodiments, this includes determining electric pulses for producing the electric fields between the selected electrodes to ablate the tissue by irreversible electroporation. In embodiments, this includes determining electric pulses for producing the electric fields between the selected electrodes to ablate the tissue by irreversible electroporation based at least in part upon the known relative locations of electrodes. Also, in embodiments, this includes determining dosing parameters for the electric fields, such as electric field strengths and the length of time that the electric field is to be applied to the cardiac tissue.

At 808, the method includes generating by a controller 90, and based on the models of the electric fields, graphical representations of the electric fields that can be produced using the selected electrodes on the catheter. In embodiments, the method includes generating the graphical representations of the electric fields based on characteristics of the catheter, the position or location of the catheter in the patient, and characteristics of the cardiac tissue surrounding the catheter in the patient.

At 810, the method includes displaying, on a display such as display 92, the graphical representations of the electric fields and the anatomical map of the patient, which can be used to aid in the planning of the ablation by electroporation and/or change ablation plans according the displayed representation in real time. In embodiments, this includes overlaying the graphical representation of the electric field of interest on the anatomical map of the heart. In embodiments, displaying the graphical representations includes displaying electric field strength that is based on electric pulse parameters of electric pulses to be provided to the selected electrodes of the electrodes.

In some embodiments, at 810, the method includes presenting, on a display, the first graphical representation of the electric fields and an anatomical map of a patient proximate to a target location. In some embodiments, at 810, the method includes presenting, on the display, the second graphical representation of the electric fields and the anatomical map proximate to the target location.

In embodiments, the graphical representation can include displaying one or more of the following: displaying at least one of electric field lines in the graphical representations of the electric field on the anatomical map, electric field strength threshold lines in the graphical representations of the electric field on the anatomical map, markings where electric field strength threshold lines intersect surrounding tissue, displaying a predicted zone of reversible electroporation, displaying a predicted zone of irreversible electroporation, displaying markings where the electric fields intersect previously created lesions, and displaying a predicted lesion on the anatomical map.

At 812, the method includes generating and displaying, a software widget (e.g. the software widget 500 in FIGS. 5A-5C) to facilitate the ablation planning. In embodiments, the software widget includes representations of one or more of the catheter, the location of the catheter, the rotation angle of the catheter, the progress of therapeutic sessions, the alignment of the catheter, and other relevant ablation information.

In some embodiments, the method may further include generating, a software widget including a second representation of the catheter and an indication of one or more therapeutic sessions of electroporation ablation conducted by the catheter. In some embodiments, the method may further include presenting the software widget on a display.

The method goes back to 800 for the next therapeutic session. Also, in embodiments, the method includes dynamically changing across therapeutic sessions, by the controller, the graphical representations of the electric fields based on one or more of changes in position of the catheter relative to surrounding tissue, changes in the catheter, changes in pulse parameters to be provided to the electrodes of the catheter; and changes in measured impedance values of the surrounding tissue.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

The present subject matter includes, inter alia, the following aspects:
1. A system for electroporation ablation, comprising:
   a catheter including an electrode assembly and a plurality of states, the electrode assembly having a first shape when the catheter is at a first state of the plurality of states, the electrode assembly having a second shape when the catheter is at a second state of the plurality of states, the second shape being different from the first shape, the electrode assembly including a plurality of electrodes;
   a controller configured to:
      generate, based on a first model of electric fields, a first graphical representation of first electric fields exceeding an electroporation threshold generated by the plurality of the electrodes when the catheter is at the first state and deployed proximate to a target location;
      generate, based on a second model of electric fields, a second graphical representation of second electric fields exceeding an electroporation threshold generated by the plurality of the electrodes when the catheter is at the second state and deployed proximate to the target location; and
      overlay, in a graphical user interface, the first graphical representations of the first electric fields and the second graphical representations of the second electric fields on an anatomical map of a patient proximate to the target location.
2. The system of aspect 1, wherein the second shape is dissimilar to the first shape.
3. The system of aspect 1 or 2, wherein the second shape is smaller than the first shape in volume.
4. The system of any one of aspects 1-3, wherein the catheter includes a catheter shaft defining a longitudinal axis, wherein the electrode assembly includes a plurality of splines, a proximal end and a distal end, wherein at least a part of the plurality of electrodes are disposed on the plurality of splines, wherein the proximal end of the electrode assembly extends from the catheter shaft.
5. The system of aspect 4, wherein each spline of the plurality of splines is arranged in a curve around the longitudinal axis and between the distal end and the proximal end of the electrode assembly when the catheter is at the first state.
6. The system of aspect 4, wherein the plurality of splines are arranged in petal-like curves when the catheter is at the second state.
7. The system of any one of aspects 1-6, wherein the controller is further configured to:
   generate an indication of a difference between the first graphical representation of the first electric fields and the second graphical representation of the second electric fields.
8. The system of any one of aspects 1-7, wherein the first graphical representation of the first electric fields includes one or more first areas, wherein field strength of the first electric fields within the one or more first areas is greater than a predefined threshold in magnitude.
9. The system of aspect 8, wherein the second graphical representation of the second electric fields includes one or more second areas, wherein field strength of the second electric fields within the one or more second area is greater than the predefined threshold in magnitude.
10. The system of any one of aspects 1-9, wherein the first graphical representation includes a first representation of the catheter.
11. The system of any one of aspects 1-10, wherein the controller is further configured to:
   generate, a software widget including a second representation of the catheter and an indication of one or more therapeutic sessions of electroporation ablation conducted by the catheter;
   present the software widget in the graphical user interface,
   wherein the software widget includes an indication identifying a therapeutic session of the one or more therapeutic sessions.
12. A method of planning ablation by electroporation, comprising;
   generating, by a controller and based on a first model of electric fields, a first graphical representation of first electric fields produced using electrodes on a catheter at a first state, the catheter including an electrode assembly having a first shape when the catheter is at the first state;
   presenting, on a display, the first graphical representation of the electric fields and an anatomical map of a patient proximate to a target location;
   generating, by a controller and based on a second model of electric fields, a second graphical representation of second electric fields produced using electrodes on a catheter at a second state, the catheter including an electrode assembly having a second shape when the catheter is at the second state, the second shape being different from the first shape; and
   presenting, on the display, the second graphical representation of the electric fields and the anatomical map proximate to the target location.
13. The method of aspect 12, wherein the second shape is dissimilar to the first shape.
14. The method of aspect 12 or 13, further comprising:
   generating an indication of a difference between the first graphical representation of the first electric fields and the second graphical representation of the second electric fields.
15. The method of any one of aspects 12-14, further comprising:
   generating, a software widget including a second representation of the catheter and an indication of one or more therapeutic sessions of electroporation ablation conducted by the catheter; and
   presenting, on the display, the software widget.

## Claims

1. A system (50) for electroporation ablation, comprising:
a catheter (105) including an electrode assembly (150) and a plurality of states, the electrode assembly (150) having a first shape when the catheter (105) is at a first state of the plurality of states, the electrode assembly (150) having a second shape when the catheter (105) is at a second state of the plurality of states, the second shape being different from the first shape, the electrode assembly (150) including a plurality of electrodes;
a controller (90) configured to:
generate, based on a first model of electric fields, a first graphical representation of first electric fields exceeding an electroporation threshold generated by the plurality of the electrodes when the catheter (105) is at the first state and deployed proximate to a target location;
generate, based on a second model of electric fields, a second graphical representation of second electric fields exceeding an electroporation threshold generated by the plurality of the electrodes when the catheter (105) is at the second state and deployed proximate to the target location;
wherein the first graphical representation includes a first inner field representation indicative of a field sufficient to perform irreversible electroporation and a first outer field representation indicative of a field sufficient to perform reversible electroporation; and
overlay, in a graphical user interface, the first graphical representations of the first electric fields and the second graphical representations of the second electric fields on an anatomical map of a patient proximate to the target location.

2. The system (50) of claim 1, wherein the second shape is smaller than the first shape in volume.

3. The system (50) of any one of claims 1-2, wherein the catheter (105) includes a catheter shaft defining a longitudinal axis, wherein the electrode assembly (150) includes a plurality of splines, a proximal end and a distal end, wherein at least a part of the plurality of electrodes are disposed on the plurality of splines, wherein the proximal end of the electrode assembly (150) extends from the catheter shaft.

4. The system (50) of claim 3, wherein each spline of the plurality of splines is arranged in a curve around the longitudinal axis and between the distal end and the proximal end of the electrode assembly (150) when the catheter (105) is at the first state.

5. The system (50) of claim 3, wherein the plurality of splines are arranged in petal-like curves when the catheter (105) is at the second state.

6. The system (50) of any one of claims 1-5, wherein the controller (90) is further configured to:
generate an indication of a difference between the first graphical representation of the first electric fields and the second graphical representation of the second electric fields.

7. The system (50) of any one of claims 1-6, wherein the first graphical representation of the first electric fields includes one or more first areas, wherein field strength of the first electric fields within the one or more first areas is greater than a predefined threshold in magnitude.

8. The system (50) of claim 7, wherein the second graphical representation of the second electric fields includes one or more second areas, wherein field strength of the second electric fields within the one or more second area is greater than the predefined threshold in magnitude.

9. The system (50) of any one of claims 1-8, wherein the first graphical representation includes a first representation of the catheter (105).

10. The system (50) of any one of claims 1-9, wherein the controller (90) is further configured to:
generate, a software widget including a second representation of the catheter (105) and an indication of one or more therapeutic sessions of electroporation ablation conducted by the catheter (105);
present the software widget in the graphical user interface,
wherein the software widget includes an indication identifying a therapeutic session of the one or more therapeutic sessions.
